(19) Europäisches Patentamt
European Patent Office
Office européen des brevets

(11) Veröffentlichungsnummer: **0 459 176 B1**

(12) **EUROPÄISCHE PATENTSCHRIFT**

(45) Veröffentlichungstag der Patentschrift: **06.09.95**

(51) Int. Cl.6: **C08G 79/02**, A61K 47/48

(21) Anmeldenummer: **91107178.5**

(22) Anmeldetag: **03.05.91**

# (54) Oligophosphate mit antiviraler Wirkung.

(30) Priorität: **16.05.90 DE 4015715**

(43) Veröffentlichungstag der Anmeldung:
**04.12.91 Patentblatt 91/49**

(45) Bekanntmachung des Hinweises auf die Patenterteilung:
**06.09.95 Patentblatt 95/36**

(84) Benannte Vertragsstaaten:
**AT BE CH DE DK ES FR GB GR IT LI LU NL SE**

(56) Entgegenhaltungen:
**EP-A- 0 386 757**
**EP-A- 0 425 268**

(73) Patentinhaber: **BAYER AG**

**D-51368 Leverkusen (DE)**

(72) Erfinder: **Löbberding, Antonius, Dr.**
**Am Rohm 105**
**W-5600 Wuppertal 1 (DE)**
Erfinder: **Heitmann, Axel Claus, Dr.**
**Auf dem Lohknippen 11**
**W-5063 Overath (DE)**
Erfinder: **Mielke, Burkhard, Dr.**
**Heisterbachstrasse 4**
**W-5090 Leverkusen 1 (DE)**
Erfinder: **Stropp, Udo, Dr.**
**Breidenhoferstrasse 12**
**W-5657 Haan (DE)**
Erfinder: **Kretschmer, Axel, Dr.**
**Richard-Zörner-Strasse 32**
**W-5060 Bergisch Gladbach (DE)**
Erfinder: **Rübsamen-Waigmann, Helga, Prof. Dr.**
**Königsteiner Strasse 113**
**W-6232 Bad Soden (DE)**
Erfinder: **Biesert, Lothar, Dr.**
**Eschersheimer Landstrasse 576**
**W-6000 Frankfurt/Main 50 (DE)**
Erfinder: **Suhartono, Haryadi, Dr.**
**Gerhart-Hauptmann-Ring 262**
**W-6000 Frankfurt/Main 50 (DE)**

EP 0 459 176 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Das gezielte Abschalten ungewollter Genexpression durch komplementäre Nukleinsäure, sogenannte Antisenseoligonukleotide, ist ein wirksames Instrument beispielsweise zur Behebung genetisch bedingter Störungen. Hierzu gehört auch das Abschalten fremder Nukleinsäuren beispielsweise nach einem Befall mit Viren, Bakterien oder Pilzen. Bei der Antisense-Technik hybridisieren Antisense-Oligonukleotidderivate mit der zu hemmenden mRNA, so daß eine Proteinsynthese ausgehend von dieser mRNA unterbunden wird. In der Literatur gibt es Belege für die Wirkung von Antisense-Oligonukleotiden.

Eine antivirale Wirkung wurde ebenfalls von Homo-Oligonukleotidphosphorothioaten sowie von Oligonukleotidphosphorothioaten mit Zufallssequenzen gefunden (Stein, C.A. et al., Aids Research and Human Retroviruses 5, 639(1989)).

In der vorliegenden Erfindung wurden überraschenderweise auch antivirale Eigenschaften von nichtnukleosidischen Oligophosphaten gefunden. Diese sind aus stark vereinfachten, kostengünstigen Bausteinen aufgebaut.

Die erfindungsgemäßen Oliogophosphate haben im Vergleich zu bekannten Oligonukleotidphosphorothioaten eine stark vereinfachte chemische Struktur:

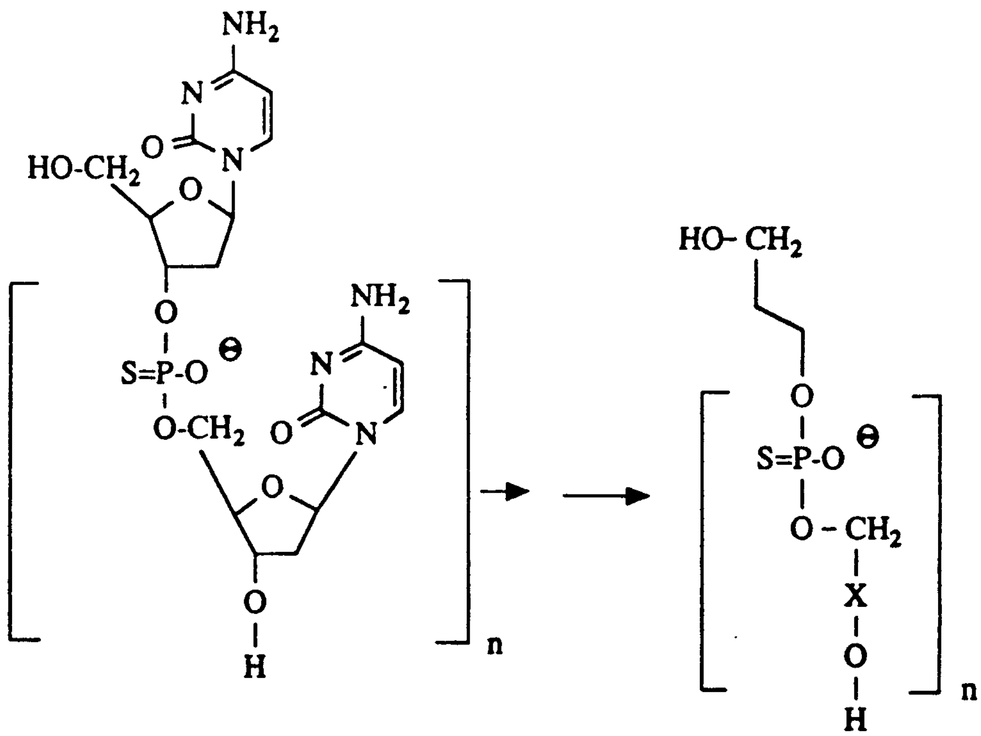

Oligonucleotidphosphorothioate Oligophosphate

wobei X und n die untengenannte Bedeutung haben.

Die vorliegende Erfindung betrifft die Oligophosphate der Formel (1)

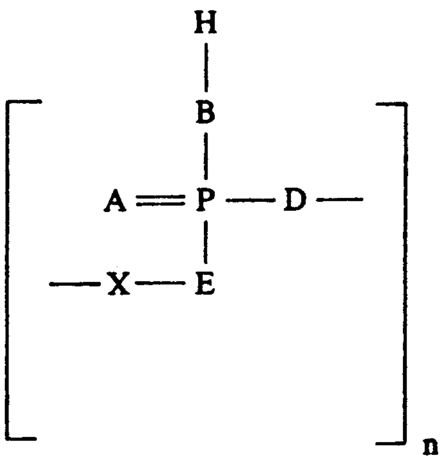

(1)

worin

n 3 - 50 sein kann,

B und E unabhängig voneinander O, S oder NH sein können,

A O, NR oder S sein kann,

D O oder S sein kann,

worin jeweils

R H, Alkyl, bevorzugt $CH_3$, $CH_2CH_3$, $CH(CH_3)_2$, Aralkyl oder Aryl, bevorzugt Benzyl oder Phenyl, sein kann,

und

X entweder $-(CH_2)_m-$ bedeutet,

worin m für 1 bis 5 steht,

oder

X

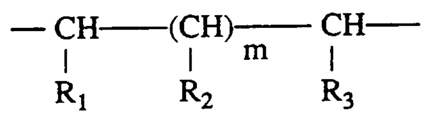

bedeutet,

worin $R_1$, $R_2$ und $R_3$ unabhängig voneinander für H, $-CH_3$, $-CH_2CH_3$, $-CH(CH_3)_2$, Phenyl, Benzyl oder $-CH_2OCH_3$ steht,

$R_2$ auch F oder Cl sein kann und

m für 0 bis 5 steht

oder

X

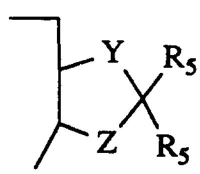

bedeutet,

worin

$R_5$ unabhängig voneinander für H, $CH_3$, $CH_2CH_3$, $CH(CH_3)_2$, Benzyl, Phenyl, $-CH_2OCH_3$, O-Aryl, O-Alkyl, H-Aryl oder N-Alkyl, O-Aralkyl, N-Aralkyl stehen,

Y für NH, O, S oder $CH_2$ steht und

Z für $-(CHR_6)_n$ steht,
wobei n 0 bis 3 sein kann und
$R_6$ die gleichen Bedeutungen wie $R_5$ haben kann,

oder

X

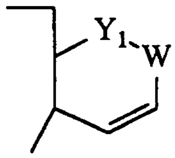

bedeutet,
worin

$Y_1$ für NR, O, S oder $CR_2$ steht,

W für $-(CH-R_7)_i-$ steht
wobei i Null oder Eins sein kann und

$R_7$ für H, OH, Alkyl, bevorzugt $CH_3$, $CH_2CH_3$, $CH(CH_3)_2$ oder Aryl, bevorzugt Benzyl, Methoxyphenyl oder
O-Alkyl, bevorzugt $O-CH_3$, $O-CH_2CH_3$, $O-CH(CH_3)_2$ oder O-Aryl, bevorzugt O-Benzyl, O-Phenyl oder
N-Alkyl, bevorzugt $N-CH_3$, $N-CH_2CH_3$, $N-CH(CH_3)_2$ oderH-Aryl, bevorzugt N-Phenyl steht.

Bevorzugt beträgt n 10 bis 30 und besonders bevorzugt 18 bis 28.

Die Herstellung der Oligophosphatbausteine erfolgte nach bekannten Verfahren und sei am Beispiel der Verbindungen 2, 3, 4, 5, 7, 8, 9 und 11 demonstriert (G. Grynkiewicz, Carbohydr. Res. 128, C9 (1984); G. Casiraghi, M. Cornia, L. Colombo, G. Rassu, G.G. Fava, M.F. Belichi, L. Zetta, Tetrahedron Lett. 29, 5549 (1988); M. Bobek, J. Kavai, E. De Clercq, J. Med. Chem. 30, 1494 (1987)).

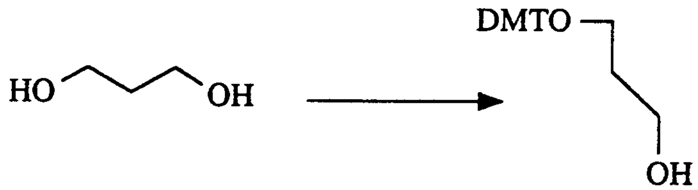

2

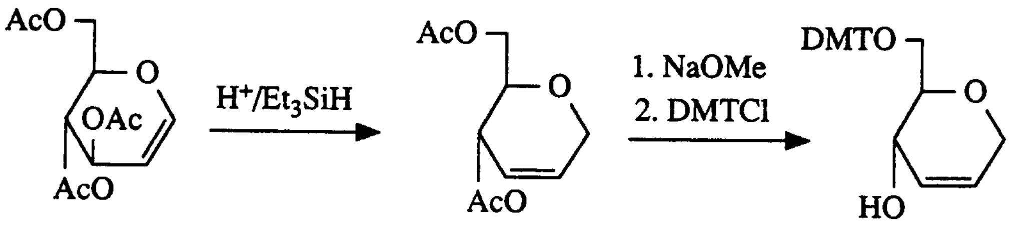
H+/Et3SiH
1. NaOMe
2. DMTCl

3

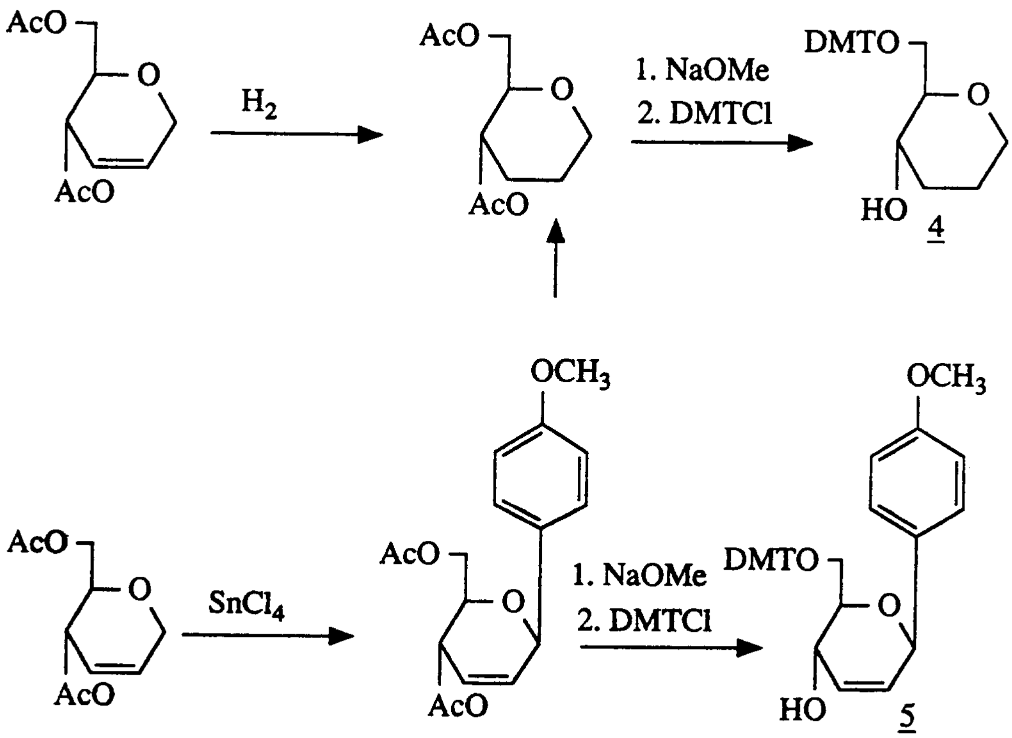
H2
1. NaOMe
2. DMTCl
SnCl4
1. NaOMe
2. DMTCl

4

5

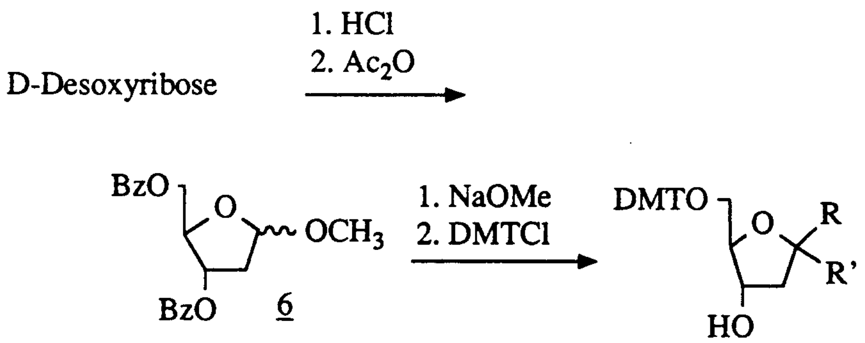


6

7 R = OMe, R' = H

8 R = H, R' = OMe

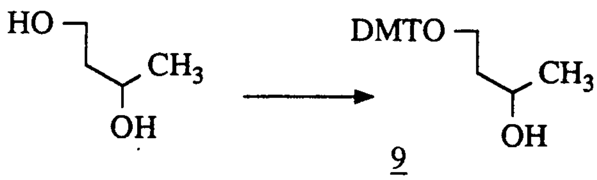

9

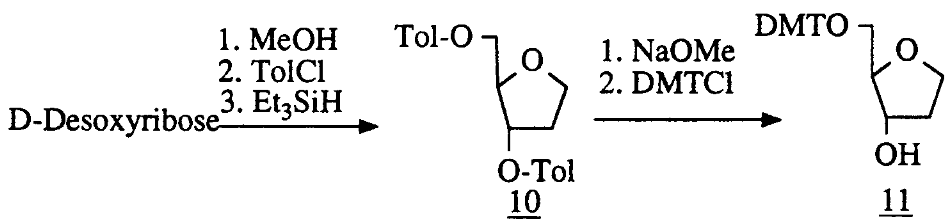


10

11

Die Dimethoxytritylierung wird in Pyridin mit Dimethoxytritylchlorid nach bekannten Verfahren durchgeführt (F. Seela, K Kaiser, Nucl. Acids Res. 15, 3113 (1987)).

Die Trennung des $\alpha/\beta$-Anomerengemisches im Falle von 6 erfolgt chromatographisch nach der Tritylierung.

Die Einführung der 1-Desoxyfunktion in 10 erfolgt durch Reduktion mit Triethylsilan (M. Takeshita, C.-N. Chang, F. Johnson, S. Will, A.P. Grollmann, J. Biol. Chem. 262 (1987), 10 171).

Verknüpfung der Oligophosphatbausteine

Die Synthese der Oligophosphate erfolgt vorzugsweise durch Festphasensynthese aber auch in flüssigen Systemen. Hierzu wurden die Vorstufen 2, 3, 4,5,7,8,9 und 11 nach bekanntem Verfahren in die Hydrogenphosphonate (B.C. Froehler, P.G. Ng, M.D. Matteucci, Nucl. Acids Res. 14, 5399 (1986)) 12,13,14,15,16,17,18 und 19 oder Phosphoramidate (S.L. Beaucage, M.H. Caruthers, Tetrahedron Lett. 22, 1859 (1981)) übergeführt und miteinander verknüpft. Die nachfolgende Oxidation erfolgt ebenfalls nach bekanntem Verfahren (W.J. Stec et al., J. Am. Chem. Soc. 106, 6077 (1984) oder P.I. Radhakrishnan et al. J. Am. Chem. Soc. 112, 1253 (1990)). Es wurden Oligophosphate mit Kettenlängen von 10 bis 50 synthetisiert.

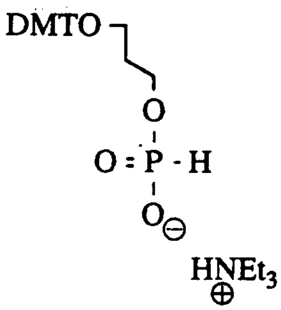

12

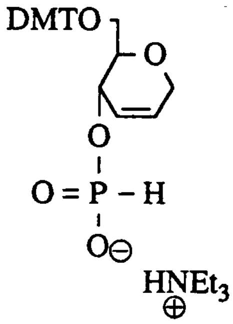

13

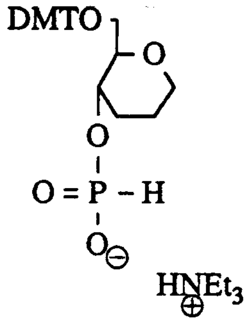

14

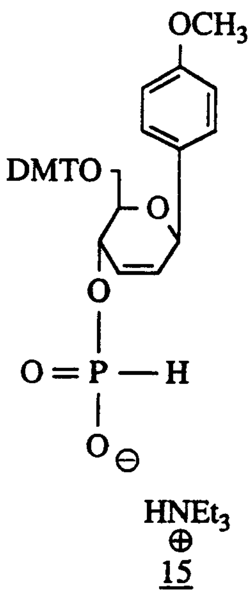

15

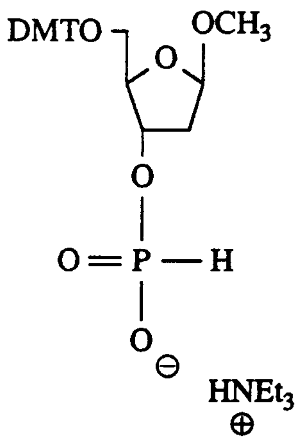

16

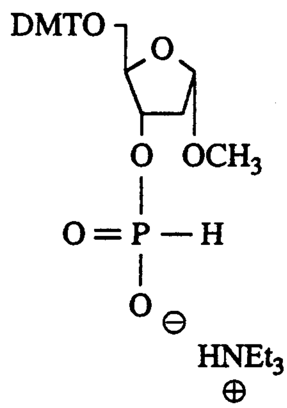

17

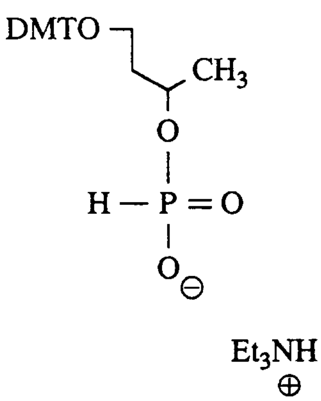

18

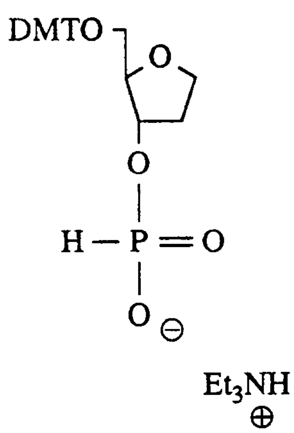

19

Synthese-Beispiele:

Beispiel 1

1-(4,4'-Dimethoxytriphenylmethoxy)-3-propanol

Zu einer Lösung von (4,4'-dimethoxytrityl)chlorid (4,9 g; 14,5 mmol) und Diisopropylamin (5,0 ml; 28,7 mmol) in trockenem Pyridin (60 ml) wird 1,3-Propandiol (5,5 g; 724 mmol) gegeben. Nach 6 Stunden bei Raumtemperatur wird mit Dichlormethan (200 ml) verdünnt, mit ges. $NaHCO_3$-Lösung und Wasser gewaschen, über $MgSO_4$ getrocknet und eingeengt. Das Rohprodukt wird chromatographisch gereinigt (Dichlormethan, 1 % Triethylamin).
Ausbeute: 4,57 g (83 %).

Beispiel 2

4,6-Di-O-acetyl-1,5-anhydro-2,3-didesoxy-D-erythrohex-2-enit

3,4,6-Tri-O-acetyl-D-glucal (9,70 g; 35,6 mmol) wurde unter Argon-Schutzgasatmosphäre in absolutem Dichlormethan (110 ml) gelöst und bei 0 °C mit Triethylsilan (4,96 g; 42,7 mmol) und Bortrifluorid-Etherat (3,04 g; 21,4 mmol) für 5 Minuten zur Reaktion gebracht. Die Reaktionslösung wurde in ges. wäßrige $NaHCO_3$-Lösung gegossen und mehrmals mit Dichlormethan extrahiert.
Die organische Phase wurde getrocknet ($MgSO_4$) und eingeengt. Das Produkt wurde durch Vakuumdestillation gereinigt.
Ausbeute: 5,87 g (77 %).

Beispiel 3

1,5-Anhydro-2,3-didesoxy-D-erythro-hex-2-enit

4,6-Di-O-acetyl-1,5-anhydro-2,3-didesoxy-D-erythro-hex-2-enit (16 g; 74,7 mmol) wurde mit 1 N Natriummethoxid (5 ml) in absolutem Methanol (50 ml) 3,5 Stunden bei Raumtemperatur deacyliert. Die Mischung wurde mit Ionenaustauscher Lewatit CNP-LF($H^+$) neutralisiert und eingeengt.
Ausbeute: 8,6 g (89 %).

Beispiel 4

1,5-Anhydro-2,3-didesoxy-6-O-(4,4'-dimethoxytriphenylmethyl)-D-erythro-hex-2-enit

1,5-Anhydro-2,3-didesoxy-D-erythro-hex-2-enit (0,5 g; 3,8 mmol) wurde mit (4,4'-Dimethoxytriphenylmethyl)chlorid (1,6 g; 4,6 mmol) und Diisopropylamin (0,8 ml; 5,7 mmol) in absolutem Pyridin (10 ml) umgesetzt. Die Aufarbeitung erfolgt analog Beispiel 1. Das Rohprodukt wurde chromatographisch gereinigt (Toluol/Ethanol 30:1, 1 % Triethylamin).
Ausbeute: 0,69 g (42 %).

Beispiel 5

4,6 Di-O-acetyl-1,5-anhydro-2,3,didesoxy-D-erythro-hexit

4,6-Di-O-acetyl-1,5-anhydro-2,3-didesoxy-D-erythro-hex-2-enit (5,17 g; 24,1 mmol) wurde in trockenem Methanol (20 ml) über Platinkohle (10 %) 25 Stunden hydriert. Filtration und Einengen ergab 4,75 g des Produkts (91 %).

Beispiel 6

1,5-Anhydro-2,3-didesoxy-D-erythro-hexit

4,6-Di-O-acetyl-1,5-anhydro-2,3-didesoxy-D-erythro-hexit (4,75 g; 22,0 mmol) wurde deacyliert wie in Beispiel 3 beschrieben.

Ausbeute: 2,8 g (97 %).

Beispiel 7

1,5-Anhydro-2,3-didesoxy-6-O-(4,4'-dimethoxytriphenylmethyl)-D-erythrohexit

1,5-Anhydro-2,3-didesoxy-D-erythro-hexit (3,0 g; 22,4 mmol) wurde mit Dimethoxytritylchlorid (9,44 g; 27,9 mmol) umgesetzt, wie in Beispiel 4 beschrieben. Die Reinigung des Rohproduktes erfolgte chromatographisch (Toluol/Ethanol 20:1,5 % Triethylamin).
Ausbeute: 4,71 g (50 %).

Beispiel 8

Methyl-2-desoxy-5-O-(4,4'-dimethoxytriphenylmethyl)-α-D-ribofuranosid und Methyl-2-desoxy-5-O-(4,4'-dimethoxytriphenylmethyl)-β-D-ribofuranosid

Zu einer Lösung von Methyl-2-desoxy-α/β-D-ribofuranosid (3,0 g; 20 mmol) in absolutem Pyridin (50 ml) wurden N,N-Dimethylaminopyridin (50 mg; 0,4 mmol) und (4,4'-Dimethoxytriphenylmethyl)chlorid (8,3 g; 24 mmol) gegeben. Die Mischung wurde 1,5 Stunden bei Raumtemperatur gerührt und danach mit Diethylether (500 ml) verdünnt, dreimal mit Wasser (je 200 ml) gewaschen, getrocknet ($MgSO_4$), in vacuo eingeengt und mehrfach mit Toluol co-destilliert. Die Trennung der Anomeren gelang durch Chromatographie an Kieselgel (Petrolether/Ethylacetat 6:1 → 1:1, jeweils 1 % Triethylamin).
Ausbeute:
3,3 g (39 %) α-Produkt
2,1 g (25 %) β-Produkt.

Beispiel 9

(±)-1-(4,4'-Dimethoxytriphenylmethoxy)-3-butanol

(±)-1,3-Butandiol (1,04 g; 11,5 mmol) wurde mit (4,4'-Dimethoxytriphenylmethyl)chlorid (3,76 g; 11,1 mmol) umgesetzt, wie in Beispiel 8 beschrieben.
Ausbeute 3,57 g /79 %).

Beispiel 10

1,2-Didesoxy-3,5-di-O-(p-toluyl)-D-ribofuranose

Zu einer Lösung von Methyl-2-desoxy-3,5-di-O-(p-toluyl)-α/β-D-ribofuranosid (5,0 g; 12 mmol) in absolutem Dichlormethan (100 ml) wurde unter Argon-Inertgasatmosphäre bei 0 °C Triethylsilan (4,0 ml; 26 mmol) und Bortrifluorid-Etherat (3,5 ml; 26 mmol) gegeben. Die Lösung wurde 1 Stunde bei 0 °C und 1 Stunde bei Raumtemperatur gerührt. Sodann wurde $NaHCO_3$-Lösung (200 ml) zugesetzt und 10 Minuten nachgerührt. Die Mischung wurde mit Dichlormethan (250 ml) verdünnt und Wasser zugesetzt (250 ml). Die organische Phase wurde abgetrennt, die wäßrige Phase 2 x mit Dichlormethan extrahiert. Die vereinigten organische Phasen wurden getrocknet ($MgSO_4$) und eingeengt. Eine Chromatographie (n-Hexan/Ethylacetat 10:1) schloß sich an.
Ausbeute: 3,7 g (80 %).

Beispiel 11

1,2-Didesoxy-D-ribofuranose

1,2-Didesoxy-3,6-di-O-(p-toluyl)-D-ribofuranose (25,0 g; 68 mmol) wurde mit 1 N Natriummethoxid deacyliert, wie in Beispiel 3 beschrieben. Das Rohprodukt wurde chromatographiert (Dichlormethan/Methanol 30:1 → 10:1).
Ausbeute: 7,4 g (92 %).

Beispiel 12

1,2-Didesoxy-5-O-(4,4'-dimethoxytriphenylmethyl)-D-ribofuranose

1,2-Didesoxy-D-ribofuranose (1,7 g; 14,4 mmol) wird dimethoxytrityliert wie in Beispiel 8 beschrieben. Das Rohprodukt wurde chromatographisch gereinigt (Toluol/Ethylacetat 9:1 → 5:1, 1 % Triethylamin).
Ausbeute: 5,4 g (89 %).

Beispiel 13

Triethylammonium-[methyl-2-desoxy-5-O-(4,4'-dimethoxytriphenylmethyl)-β-D-ribofuranosid-3-O-hydrogenphosphonat]

1,2,4-Triazol (5,3 g; 76,7 mmol) wurde unter Argon-Inertgasatmosphäre in absolutem Dichlormethan (220 ml) gelöst. N-Methylmorpholin (25,6 ml; 233 mmol) wurde zugegeben und die Lösung auf 0 °C gekühlt. Phosphortrichlorid (2,1 ml; 24 mmol) wurde zugesetzt, 30 Minuten bei Raumtemperatur gerührt, wiederum auf 0 °C gekühlt und eine Lösung von Methyl-2-desoxy-5-O-(4,4'-dimethoxytriphenylmethyl)-β-D-ribofuranosid (2,1 g; 4,7 mmol) in absolutem Dichlormethan (70 ml) innerhalb 60 Minuten zugetropft. Nach 90 Minuten bei Raumtemperatur wurde die Reaktionsmischung in ges. Triethylammoniumhydrogencarbonat-Lösung (900 ml) gegossen. Die organische Phase wurde abgetrennt, 2 x mit Wasser gewaschen, getrocknet ($MgSO_4$) und eingeengt Das Rohprodukt wurde an Kieselgel chromatographiert (Toluol/Ethanol 1:1, 1 % Triethylamin).
Ausbeute: 2,1 g (73 %).

Beispiel 14

Triethylammonium-[methyl-2-desoxy-5-O-(4,4'-dimethoxytriphenylmethyl)-α-D-ribofuranosid-3-O-hydrogenphosphonat]

Methyl-2-desoxy-5-O-(4,4'-dimethoxytriphenylmethyl)-α-D-ribofuranosid (3,3 g; 7,3 mmol) wurde in das Hydrogenphosphonat überführt, wie in Beispiel 13 beschrieben.
Ausbeute: 3,2 g (71 %).

Beispiel 15

Triethylammonium-[1,2-didesoxy-5-O-(4,4'-dimethoxytriphenylmethyl)-D-ribofuranose-3-O-hydrogenphosphonat]

1,2-Didesoxy-5-O-(4,4'-dimethoxytriphenylmethyl)-D-ribofuranose (2,0 g; 4,8 mmol) wurde in das Hydrogenphosphonat überführt, wie in Beispiel 13 beschrieben.
Ausbeute: 2,4 g (85 %).

Beispiel 16

Triethylammonium-[(±)-1-(4,4'-dimethoxytriphenylmethoxy)-3-butanol-3-O-hydrogenphosphonat]

(±)-1-(4,4'-Dimethoxytriphenylmethoxy)-3-butanol(3,5 g; 6,3 mmol) wurde umgesetzt, wie in Beispiel 13 beschrieben.
Ausbeute: 3,3 g (94 %).

Beispiel 17

Triethylammonium-[1-(4,4'-dimethoxytriphenylmethoxy)-3-propanol-3-O-hydrogenphosphonat]

1-(4,4'-Dimethoxytriphenylmethoxy)-3-propanol (3,8 g; 10,0 mmol) wurde umgesetzt, wie in Beispiel 13 beschrieben.
Ausbeute: 4,5 g (82 %).

Beispiel 18

Triethylammonium-[1,5-anhydro-2,3-didesoxy-6-O-(4,4'-dimethoxytriphenylmethoxy)-D-erythro-hexit-4-O-hydrogenphosphonat]

1,5-Anhydro-2,3-didesoxy-6-O-(4,4'-dimethoxytriphenylmethyl)-D-erythro-hexit (6,35 g; 15,1 mmol) wurde umgesetzt, wie in Beispiel 13 beschrieben.
Ausbeute: 7,63 g (84 %).

Beispiel 19

Synthese eines Oligophosphates mit der nachfolgend aufgeführten Struktur:

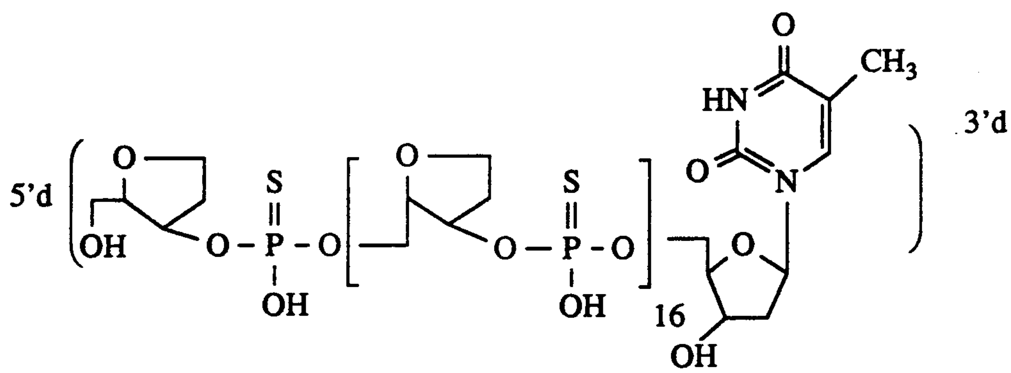

(Allgemeines Verfahren)

Die Synthese der Oligophosphate wurde an einem Applied Biosystems 380 B DNA-Syntheseautomaten durchgeführt und mit einer kommerziell erhältlichen (Applied Biosystems) 10 µmol control pore glass Thymidinsäule begonnen. Die 4,4'-Dimethoxytrityl-Schutzgruppe (DMT) wurde von der 5'-Hydroxyfunktion des Thymidinbausteins mit 2,5 %iger Dichloressigsäure in Methylenchlorid abgetrennt. Überschüssige Dichloressigsäure wird mit Acetonitril ausgewaschen. Eine 0,5 M Lösung von Triethylammonium[1,2-didesoxy-5-O-(4,4'-dimethoxy-trityl)-D-ribofuranose-3-O-hydrogenphosphonat] in trockenem Acetonitril/Pyridin 1:1 wird mit einer 0,5 M Lösung von 1-Adamantancarbonylchlorid in Acetonitril/Pyridin 1:1 80 Sekunden aktiviert und dann zur Reaktionssäule gegeben.

Nach 120 Sekunden wird überschüssiges Reagenz mit Acetonitril/Pyridin (1:1) ausgewaschen. Die capping-Reaktion wurde gemäß dem Applied Biosystems user bulletin Nr. 44 (1987) durchgeführt: Hierzu wurde eine äquimolare Lösung von Isopropylphosphit und 1-Adamantancarbonylchlorid zur Reaktionssäule gegeben. Überschüssiges Reagenz wird im Anschluß mit Acetonitril ausgewaschen.

Der nächste Reaktionszyklus beginnt wieder mit der Abspaltung der 4,4'-Dimethoxytritylschutzgruppe durch Behandlung mit 2,5 %iger Dichloressigsäure usw. Entsprechend wird der Synthesezyklus 16 mal wiederholt.

Die Einführung von Schwefel erfolgt unabhängig von Syntheseautomaten durch Behandlung des Hydrogenphosphonatintermediates mit einer Lösung von $S_8$ in Schwefelkohlenstoff/Pyridin (1:1) für eine Stunde. Überschüssiges Reagenz wird abgetrennt durch Waschen mit Schwefelkohlenstoff/Pyridin.

Das Oligophosphat wird vom polymeren Träger durch Behandlung mit konzentriertem Ammoniak für 1 Stunde abgetrennt. Dann wird das Produkt durch HPLC an einer RP 18-Säule mit einem in 30 Minuten linear ansteigenden Gradienten von Acetonitril in 0,1 M Triethylammoniumacetat abgetrennt.

Die Tritylschutzgruppe (DMT) wird durch Behandlung mit 80 %igem Eisessig bei Raumtemperatur für 30 Minuten abgetrennt. Das Oligophosphat wird anschließend durch nochmalige HPLC an einer RP 18-Säule - wie bereits zuvor beschrieben - isoliert.

Antivirale Aktivität von Oligophosphaten

Oligophosphate sind aktiv gegen Retroviren, inbesondere gegen das HIV-Virus.

Die Prüfung der Oligophosphate wurde an den nachfolgend aufgeführten Virus Isolaten durchgeführt:

$HIV\text{-}1_{K31,Zaire}$ Isolierung durch Dr. von Briesen und Prof. H. Rübsamen-Waigmann, Georg-Speyer-Haus, Frankfurt 1987

$HIV\text{-}2_{ROD}$ Isolierung durch Pasteur Institut Paris 1986

Als Testsystem dienten Nabelschnurlymphozyten von Neugeborenen, die mit Phytohämagglutinin voraktiviert wurden. Die Substanzen wurden unmittelbar nach der Infektion zur Zellkultur gefügt. In der nachfolgenden Tabelle sind die Konzentrationen aufgeführt, bei der keine Synzytienbildung mehr erkannt wurde.

Antivirale Eigenschaften von Oligophosphaten

| Verbindung | Baustein | HIV 1 µg/ml | µmol | HIV 2 µg/ml | µmol |
|---|---|---|---|---|---|
| | | Angereicherte | | CD4-Lymphozyten | |
| 20 | 12 | 5-10 | 2-4 | 5-10 | 2-4 |
| 21 | 13 | 5-10 | 1,6-3,2 | 5-10 | 1,6-3,2 |
| 22 | 14 | - | - | 5-10 | 1,5-0,3 |
| 23 | 19 | - | - | 0,75 | 0,2 |

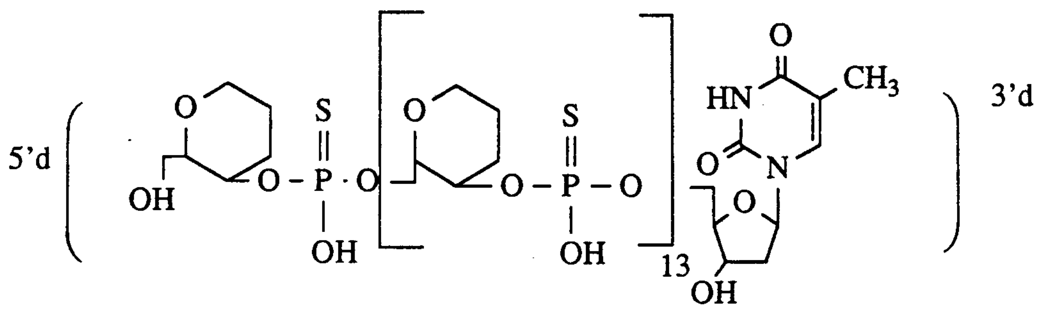

22

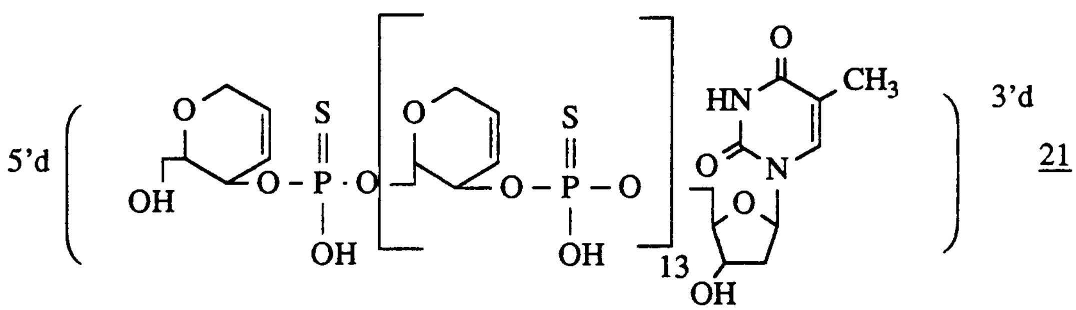

21

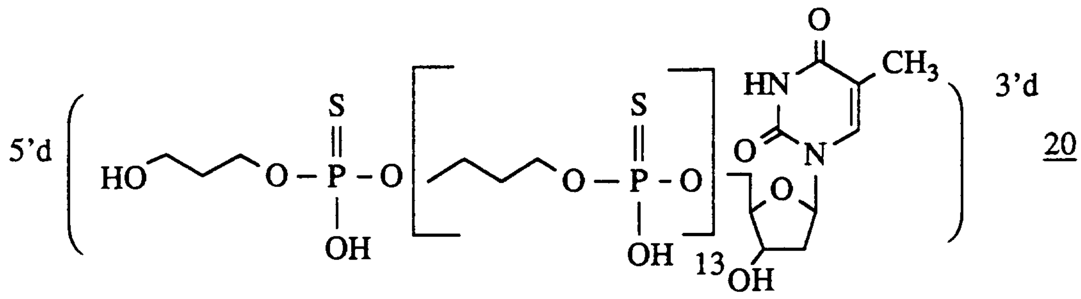

20

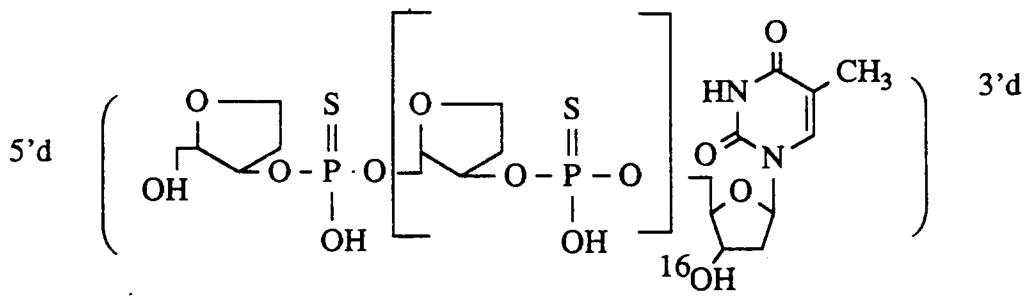

23

Die entsprechenden pharmazeutischen Zubereitungen enthalten neben den Oligophosphaten die für parenterale Zubereitungen üblichen Hilfsstoffe wie z.B. Puffer und/oder Stabilisatoren oder Liposomenformulierungen. Ferner ist eine topische Anwendung denkbar. Die hierfür einsetzbaren Zubereitungen sind z.B. Salben, Cremes, Lösungen oder Pflaster, die neben dem Wirkstoff die für diese Anwendung geeigneten pharmazeutischen Hilfsstoffe enthalten.

## Patentansprüche

**1.** Oligophosphate der Formel (1)

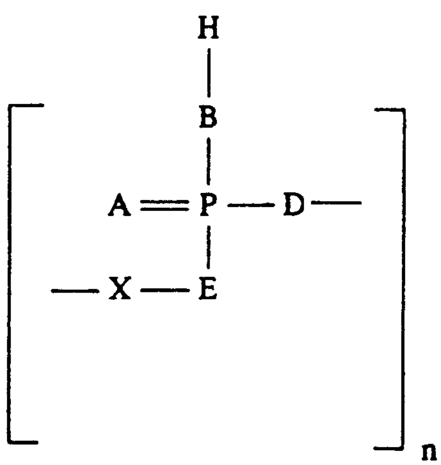

(1)

worin

| | |
|---|---|
| n | 3-50 sein kann, |
| B und E | unabhängig voneinander O, S oder NH sein können, |
| A | O, NR oder S sein kann, |
| D | O oder S sein kann<br>worin jeweils |
| R | H, Alkyl oder Aryl sein kann, |

und

X entweder $-(CH_2)_m-$ bedeutet, worin m für 1 bis 5 steht,

oder

X

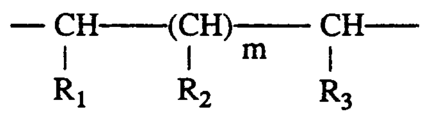

bedeutet,

worin $R_1$, $R_2$ und $R_3$ unabhängig voneinander für H, $-CH_3$, $-CH_2CH_3$, $-CH(CH_3)_2$, Benzyl, Phenyl oder $-CH_2OCH_3$ stehen,

$R_2$ auch F oder Cl sein kann und

m für 0 bis 5 steht,

oder

X

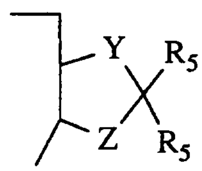

bedeutet,

worin

$R_5$ unabhängig voneinander für H, $CH_3$, $CH_2CH_3$, $CH(CH_3)_2$, Benzyl, Phenyl, $-CH_2OCH_3$, O-Aryl, O-Alkyl, H-Aryl oder H-Alkyl stehen und

Y für NH, O, S oder $CH_2$ steht und

Z für $-(CHR_6)_n$ steht,

wobei n 0 bis 3 sein kann

$R_6$ die gleichen Bedeutungen wie $R_5$ haben kann

oder

X

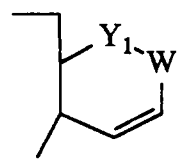

bedeutet, worin

$Y_1$ für N R, O, S, $CR_2$ steht,

W für $-(CH-R_7)_i-$ steht,

wobei i Null oder Eins sein kann und

$R_7$ für H, OH, Alkyl, Aryl, O-Alkyl, O-Aryl, H-Alkyl oder H-Aryl steht.

**2.** Oligophosphate nach Anspruch 1, wobei n 10 bis 30 beträgt

**3.** Oligophosphate nach Anspruch 1, wobei n 18 bis 28 beträgt.

**4.** Arzneimittel enthaltend eins oder mehrere der acyclischen Oligophosphate der Ansprüche 1 bis 3.

**5.** Verwendung der Oligophosphate der Ansprüche 1 bis 3 zur Herstellung von Arzneimitteln.

**Claims**

**1.** Oligophosphates of the formula (1)

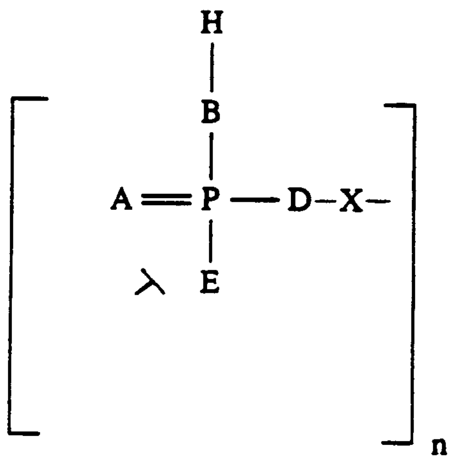

(1)

in which

n can be 3-50,
B and E can be, independently of one another, O, S or NH,
A can be O, NR or S,
D can be O or S,
in each of which
R can be H, alkyl or aryl,

and

X either denotes $-(CH_2)_m-$
in which m represents 1 to 5,

or

X denotes

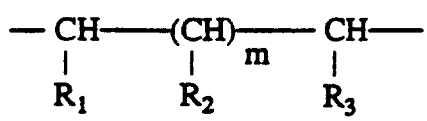

in which $R_1$, $R_2$ and $R_3$ represent, indepen-$CH(CH_3)_2$, benzyl, phenyl or $-CH_2OCH_3$,
$R_2$ can also be F or Cl, and
m represents 0 to 5,

or

X denotes in which

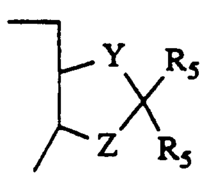

$R_5$ represent, independently of one another, H, $CH_3$, $CH_2CH_3$, $CH(CH_3)_2$, benzyl, phenyl, $-CH_2OCH_3$, O-aryl, O-alkyl, H-aryl or H-alkyl, and

Y represents NH, O, S or $CH_2$, and
Z represents $-(CHR_6)_n$,
where n can be 0 to 3, and
$R_6$ can have the same meanings as $R_5$,

or

X denotes

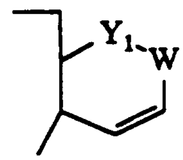

in which
Y represents NR, O, S, $CR_2$,
W represents $-(CH-R_7)_i-$
where i can be zero or one, and
$R_7$ represents H, OH, alkyl, aryl, O-alkyl, O-aryl, H-alkyl or H-aryl.

**2.** Oligophosphates according to Claim 1, where n is 10 to 30.

**3.** Oligophosphates according to Claim 1, where n is 18 to 28.

**4.** Medicament containing one or more of the acyclic oligophosphates of Claims 1 to 3.

**5.** Use of the oligophosphates of Claims 1 to 3 for preparing medicaments.

**Revendications**

**1.** Oligophosphates de formule (1)

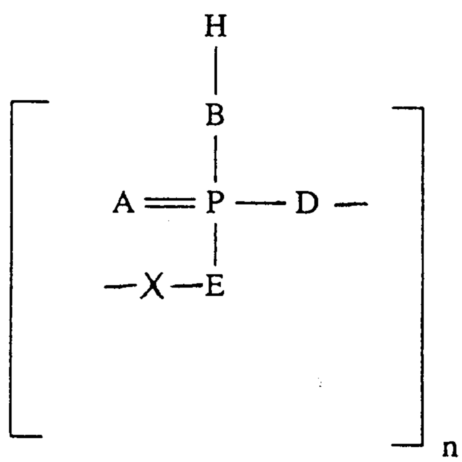

(1)

dans laquelle
n peut avoir une valeur de 3 à 50,
B et E peuvent représenter, indépendamment l'un de l'autre O, S ou NH,
A peut représenter O, NR ou S,
D peut représenter O ou S,
R pouvant représenter dans chaque cas H, un groupe alkyle ou un groupe aryle,

et

X représente un groupe $-(CH_2)_m-$,
dans lequel m a une valeur de 1 à 5,

ou bien

X représente un groupe

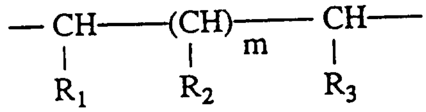

dans lequel $R_1$, $R_2$ et $R_3$ représentent, indépendamment les uns des autres, H, un groupe $-CH_3$, $-CH_2CH_3$, $-CH(CH_3)_2$, benzyle, phényle ou $-CH_2OCH_3$,

$R_2$ peut aussi représenter F ou Cl et

m a une valeur de 0 à 5,

ou bien

X représente un groupe

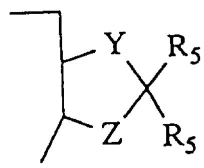

dans lequel

les substituants $R_5$ représentent, indépendamment l'un de l'autre, H, un groupe $CH_3$, $CH_2CH_3$, $CH(CH_3)_2$, benzyle, phényle, $-CH_2OCH_3$, O-aryle, O-alkyle, H-aryle ou H-alkyle,

Y représente NH, O, S ou $CH_2$ et

Z est un groupe $-(CHR_6)_n$,

dans lequel n peut avoir une valeur de 0 à 3, $R_6$ peut avoir les mêmes définitions que $R_5$,

ou bien

X est un groupe

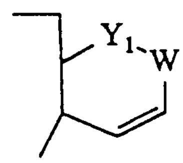

dans lequel

$Y_1$ représente NR, O, S, $CR_2$,

W est un groupe $-(CH-R_7)_i-$,

où i peut être égal à zéro ou à un et

$R_7$ représente H, un groupe OH, alkyle, aryle, O-alkyle, O-aryle, H-alkyle ou H-aryle.

**2.** Oligophosphates suivant la revendication 1, dans lesquels n a une valeur de 10 à 30.

**3.** Oligophosphates suivant la revendication 1, dans lesquels n a une valeur de 18 à 28.

**4.** Médicament contenant un ou plusieurs des oligophosphates acycliques suivant les revendications 1 à 3.

**5.** Utilisation des oligophosphates suivant les revendications 1 à 3 pour la préparation de médicaments.